# EUROPEAN PATENT APPLICATION

(11) **EP 1 862 534 A1**
(43) Date of publication of application: **05.12.2007**
(21) Application number: 07010900.4
(22) Date of filing: 01.06.2007
(51) Int. Cl.: C12M 1/34, C12M 1/42

(54) **Microorganism separation system and method**

(30) Priority: 02.06.2006 JP 2006154141
(71) Applicant: Hitachi Plant Technologies, Ltd., Chiyoda-ku Tokyo 101-0047 (JP)
(72) Inventor: Sano, Tadashi, Tokyo 100-8220 (JP); Sasaki, Yasuhiko, Tokyo 100-8220 (JP); Ikuta, Hajime, Tokyo 101-0047 (JP); Isaka, Kazuichi, Tokyo 101-0047 (JP); Sumino, Tatsuo, Tokyo 101-0047 (JP); Togashi, Shigenori, Tokyo 100-8220 (JP); Miyamoto, Tetsuro, Tokyo 100-8220 (JP)
(74) Representative: Beetz & Partner

(57) **Abstract**

The invention relates to a method for separating microorganisms dispersed in a sample solution in order to recover specific microorganisms or strains, and to a microorganism separation system comprising a sample solution container (34) containing microorganisms, a separator (1), a plurality of receivers (47), designed to separate microorganisms from the sample solution, a microorganism detection sensor (55, 7, 8) and a plate (49) which has the plurality of receivers (47) connected to each other, and an identification indicator (48), wherein when the microorganism detection sensor (55, 7, 8) determines that a microorganism has passed, the supply of the sample solution is stopped, the detected microorganism is discharged together with the sample solution, and then the solution is injected into a receiver (47). During the time period from the start of the injection to the end, the number of times microorganisms are detected is recognized as separation quantity, and then, as separation information, are stored: the separation quantity, the number of a receiver (47) into which a microorganism was injected for each identification indicator (48), the signal waveform sent from the microorganism detection sensor (55, 7, 8) at the separation, and date and temperature.

## Description

### BACKGROUND OF THE INVENTION

### [Field of the Invention]

The present invention relates to a microorganism separation system and method for separating microorganisms which are dispersed in a sample solution, and depositing them into a container for use.

### [Prior Art]

Microorganisms (including bacteria) are effectively used in a variety of fields such as food and beverage manufacturing and wastewater treatment. Microorganisms are classified according to their characteristics, for example, lactic acid bacteria, and coliform bacteria; and those microorganisms or bacteria can be further classified into subcategories. Even if microorganisms belong to the same group, their ability is different so they are further classified into subcategories; therefore, when using microorganisms, it is necessary to use microorganisms best suitable for a specific purpose. Accordingly, it is necessary to classify microorganisms, which belong to each group, into the level of the "strain" which is the finest classification, and then to compare the abilities of each "strain."

When separating microorganisms into "strains," or separating one "strain", the separation procedure is usually conducted manually by a serial dilution method. Furthermore, a well-known apparatus for measuring particles, such as microorganisms, is the particle analyzing apparatus described in the Japanese Patent Application Laid-open publication No. 2000-74816 (Patent Document 1), and so forth. Moreover, a well-known apparatus for automatically separating microorganisms is the flow cytometry apparatus wherein a ray of light is irradiated to a sample solution that contains microorganisms, the type of microorganisms contained in the solution is evaluated, and desired microorganisms are obtained by means of a downstream separation mechanism. For example, the apparatus is described in the Japanese Patent Application Laid-open publication No. Hei 09(1997)-145593 (Patent Document 2).

### SUMMARY OF THE INVENTION

Since it is not possible to detect and separate living microorganisms by using the above-mentioned conventional technology, separated microorganisms cannot be used effectively. Furthermore, the conditions under which the microorganisms were obtained are not clearly known; therefore, it is difficult to understand the nature and properties of those microorganisms.

It is the underlying problem of the present invention to solve the problems of the above-mentioned conventional technology, thereby making it possible to efficiently separate living microorganisms contained in a sample solution so as to make use of them. Furthermore, the problem to be solved of the present invention is also to provide a microorganism separation system which consistently conducts separation, transportation, and utilization procedures as well as facilitates management, thereby enabling the efficient and effective use of microorganisms, and to provide a corresponding microorganism separation method.

The above problems are solved according to the independent claims. The dependent claims relate to preferred embodiments of the concept of the present invention.

To achieve the above-mentioned purpose, the present invention provides a microorganism separation system comprising, as a separating apparatus,
a sample solution container containing a sample solution including microorganisms,
a separator having a first flow channel and a second flow channel,
a pump for supplying the sample solution in the sample solution container to the first flow channel,
a carrier liquid container containing a carrier liquid,
a carrier liquid pump for supplying the carrier liquid in the carrier liquid container to the second flow channel, and
a receiver for receiving a carrier liquid containing the sample solution discharged from the end portion of the second flow channel, designed to separate microorganisms dispersed in the sample solution; further comprising
a microorganism detection sensor located between the end portion of the first flow channel and the second flow channel for emittig a detection signal when a microorganism passes, and
a plate having a plurality of the receivers connected to each other and an identification indicator, wherein
when the microorganism detection sensor determines that a microorganism has passed, supply of the sample solution to the first flow channel is stopped, the detected microorganism is discharged together with the sample solution from the end portion of the second flow channel, and then the solution starts to be injected into the receiver; and, the number of times microorganisms are detected during the time period from the start of the injection to the end is recognized as separation quantity; and then as separation information, the separation quantity, and/or the number for the receiver into which a microorganism was injected for each identification indicator, signal waveform sent from the microorganism detection sensor at the separation procedure, date and temperature are stored.

The method of the invention for separating microorganisms dispersed in a sample solution in a microorganism separation system comprising a sample solution container containing the sample solution including microorganisms,
a separator having a first flow channel and a second flow channel,
a pump for supplying the sample solution in the sample solution container to the first flow channel,
a carrier liquid container containing a carrier liquid,
a carrier liquid pump for supplying the carrier liquid in the carrier liquid container to the second flow channel, and
a receiver for receiving the carrier liquid containing the sample solution discharged from the end portion of the second flow channel, designed to separate microorganisms dispersed in the sample solution,
a microorganism detection sensor located between the end portion of the first flow channel and the second flow channel and emitting a detection signal when a microorganism passes, and
a plate having a plurality of the receivers connected to each other and an identification indicator,
is characterized by:
   - introducing the sample solution from the sample solution container into the first flow channel of the separator,
   - continuously monitoring a physical property of the sample solution with the microorganism detection sensor, preferably the electrical resistance,
   - detecting the passage of a microorganism through an orifice at the end of the first flow channel by a change of the signal of the microorganism detection sensor,
   - stopping the supply of sample solution by stopping the pump if a microorganism has passed through the orifice and resides in the second flow channel, and
   - operating the carrier liquid pump and injecting the carrier liquid containing the microorganism into a receiver,
   - repeating these steps for injecting carrier liquid containing microorganisms one by one into a plurality of containers, and
   - detecting the number of times microorganisms have been detected during the time period from the start of the introduction of the sample solution to the stopping of the sample solution supply to the first flow channel, which corresponds to the number of separated microorganisms that were dispensed into a respective receiver (separation quantity),
   - rejecting containers containing mor than one microorganism or contain impurities smaller than a microorganism by checking the signal waveforms detected at the respective separation operation, and using one container containing only one single microorganism for analysis and/or recovery.

According to the present invention, it is possible to efficiently separate living microorganisms contained in a sample solution so as to make use of them and also consistently conduct separation, transportation, and utilization procedures.

### BRIEF DESCRIPTION OF THE DRAWINGS

FIG. 1 is a schematic diagram to show a separator according to an embodiment of the present invention.

FIG. 2 is a schematic diagram to show a separating apparatus according to the embodiment of the present invention.

FIG. 3 is a system configuration diagram according to the embodiment of the present invention.

FIG. 4 is a processing flow chart to show an operation of the embodiment of the present invention.

FIG. 5 shows the analysis quantity determination screen according to the embodiment of the present invention.

FIG. 6 shows the recovery quantity determination screen according to the embodiment of the present invention.

### DETAILED DESCRIPTION OF THE INVENTION

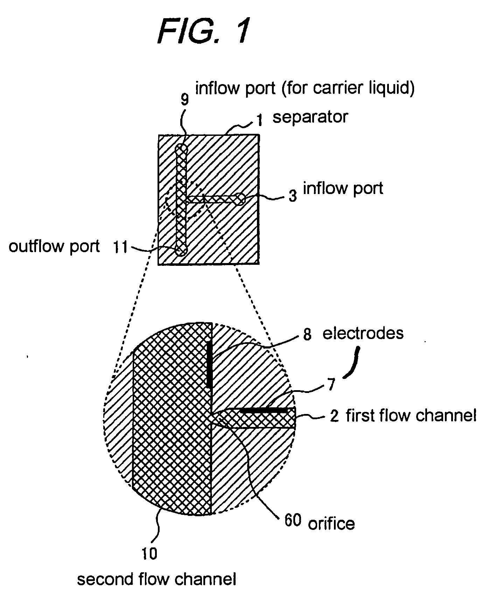

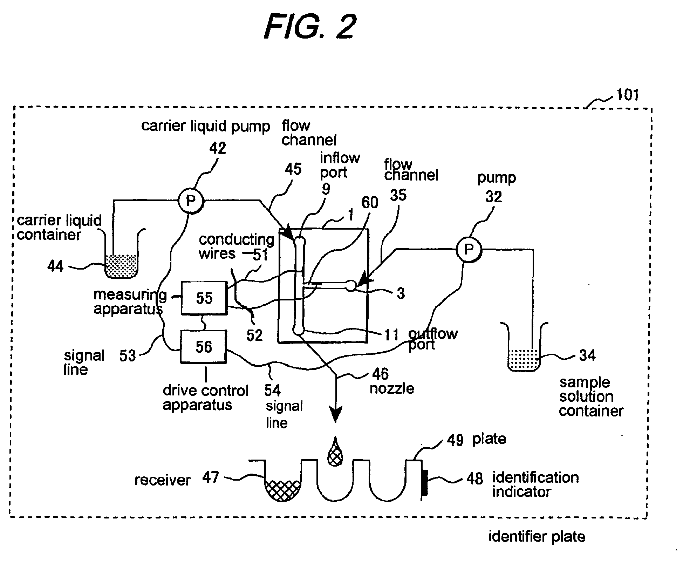

FIG. 1 and FIG. 2 are schematic diagrams of a separating apparatus which is part of a microorganism separation system that is an embodiment of the present invention. The microorganism separating apparatus, shown in FIG. 1 and FIG. 2, comprises
a separator 1, an inflow port 3 for injecting bacteria solution into a first flow channel 2, a pump 32 for supplying the bacteria solution, a sample solution container 34 in which the bacteria solution is reserved, a flow channel 35 for supplying a liquid which contains microorganisms from the sample solution container 34, a second flow channel 10 for ejecting microorganisms sent from the first flow channel 2 by means of a carrier liquid, an inflow port 9 for injecting the carrier liquid into the second flow channel 10, a carrier liquid pump 42 for supplying the carrier liquid, a carrier liquid container 44 in which the carrier liquid is stored, a flow channel 45 for supplying carrier liquid from the carrier liquid container 44, an outflow port 11 for directing the liquid flowing through the second flow channel 10 to the outside, and a nozzle 46 connected to the outflow port 11.

Furthermore, the microorganism separating apparatus further comprises a receiver 47 for receiving liquid discharged from the nozzle 46, a plate 49 which comprises a plurality of receivers 47 connected to form one container, an identification indicator 48, such as barcode or IC tag, to identify the plate, a measuring apparatus 55 for measuring the resistance between electrodes 7 and 8 which function as microorganism detection sensor, conducting wires 51 and 52 which connect the measuring apparatus 55 to the electrodes 7, 8, a drive control apparatus 56 for controlling the drive of pumps 32 and 42, and signal lines 53 and 54 for transmitting drive signals.

For the preparation to discharge a carrier liquid, the carrier liquid is filled by a carrier liquid pump 42, and then a bacteria solution containing microorganisms is discharged from the first flow channel 2 by the pump 32. At this time, resistance between electrodes 7 and 8 is continuously measured, and the section at which the first flow channel 2 merges with the second flow channel 10 is a small orifice 60 whose flow channel cross-sectional area is small. When a microorganism passes through the orifice 60, the value of resistance between electrodes 7 and 8 changes. Changes of the resistance are detected by the measuring apparatus 55, and when the system judges that a microorganism has passed, the drive control apparatus 56 stops the operation of the pump 32. The microorganism that has passed resides in the second flow channel 10, and the carrier liquid pump 42 is driven in the direction of discharge so as to send the microorganism to the receiver 47 and start the injection procedure. The carrier liquid containing the microorganism passing through the nozzle 46 is injected into one of arbitrary receivers 47. Furthermore, by moving the separator 1 to a position above another receiver 47 and then repeating the operation, it is possible to continuously inject microorganisms one by one into a plurality of containers 47.

When the carrier liquid pump 42 is driven in the direction of discharge, microorganisms, which should reside in the first flow channel 2, pass through the orifice 60, and microorganisms are detected during the period from the start of injection to the end of the injection; consequently, two or more microorganisms including the one that is to be originally dispensed are sometimes dispensed into the receiver 47. As a result, the number of times the microorganism detection sensor judges that microorganisms have passed through indicates the number of separated microorganisms that were dispensed into the receiver 47.

When the bacteria concentration of the prepared bacteria solution 34 (sample solution container) is low, even if the bacteria solution is continuously supplied by the pump 32, microorganisms would not easily pass through the orifice 60. Therefore, only the liquid of the bacteria solution drops from the nozzle 46, thereby decreasing the injectable volume of the receiver 47. And, when dispensing microorganisms, there is a lower limit to the necessary amount of the bacteria solution discharged by the carrier liquid pump 42; therefore, the injectable volume is lower than the lower-limit value, decreasing the amount of dispensed microorganisms by that volume. Therefore, the separation quantity in the receiver 47 is zero.

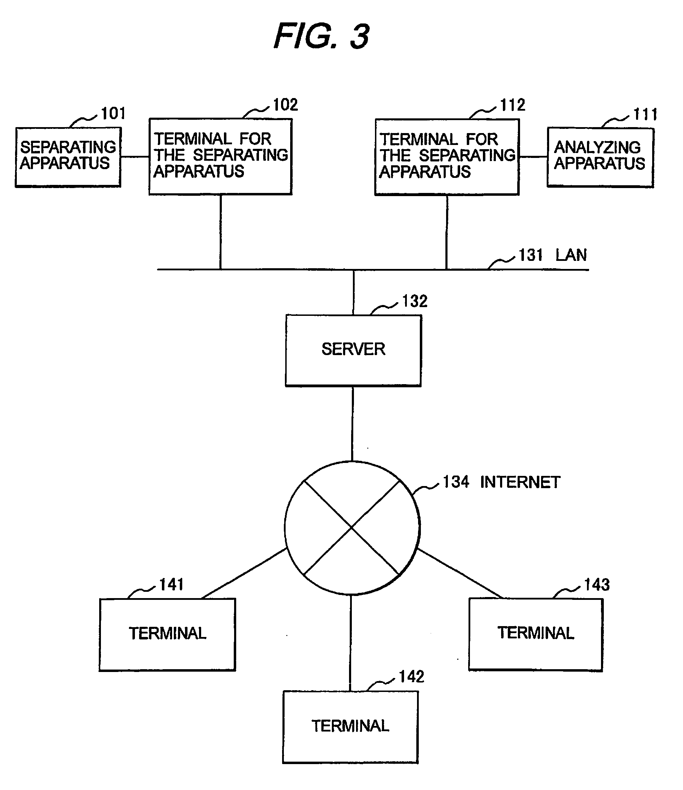

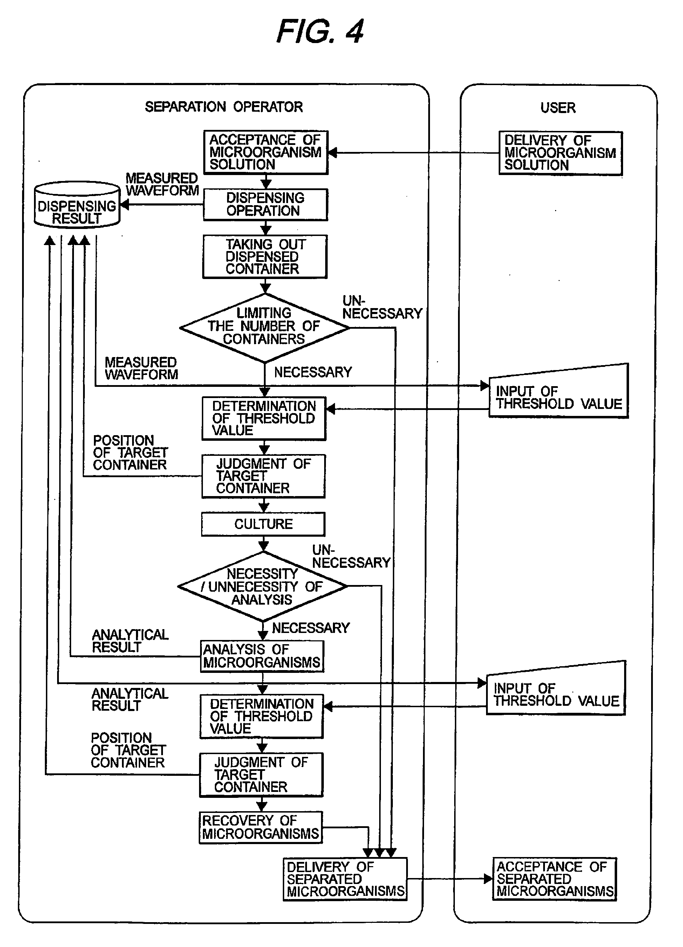

FIG. 3 shows a microorganism separation system, and FIG. 4 is a flowchart to show the operation of the microorganism separation system. Signals emitted by a sensor located in the separating apparatus 101 are stored in the server 132 via a terminal 102 for the separating apparatus. Furthermore, the result values measured by the analyzing apparatus 111 are stored in the server 132 via a terminal 112 for the separating apparatus. The microorganism separation system allows the system users to access the server 132 from each user's terminal 141, 142, or 143 via the Internet, browse the separation result and the analytical result and enter instructions. Furthermore, the separation result is the number of microorganisms, while the analytical result is the data obtained by investigating the existence of microorganisms, measuring the absorbance, fluorescence degree, pH, oxidation-reduction potential, COD, BOD, TOC dissolved oxygen concentration of the contents of the culture vessel, or staining a microorganism by a reagent and measuring the absorbance and the fluorescence degree. Moreover, it is possible to directly measure metabolite of the microorganism or indirectly measure it by using a reagent etc.

A system user prepares a microorganism solution (sample solution) in which two or more types of microorganisms coexist and hands it over to a separation operator. At this time, data about necessity or unnecessity of culture and analysis is stored in the server 132. The operator will conduct separation operation of the microorganism solution by using a separating apparatus 101. Every time a microorganism is separated, a waveform is obtained from a sensor, data of the identification indicator 48 attached to a plate 49 is automatically read, and the waveform sent from the sensor as well as a number for the receiver 47 in which microorganisms are injected and the waveform, date, and temperature obtained at the separation operation are stored in the server 132. Depending on the operation of the separating apparatus 101, the number of microorganisms injected into the receiver 47 is zero, one, or more than one, and separation information including separation quantity information is stored.

When culture is not necessary, an operator delivers the separated containers to the user. The user accesses the server 132, enters the container number described on the container or uses a reading apparatus to read the identification indicator, and can browse separation quantity information which is the container's separation result. Based on the separation quantity information stored in the server, containers other than the container which contains only one microorganism, that is, the containers which contain no microorganism or contain two or more microorganisms are indicated as being exempt from analysis and recovery.

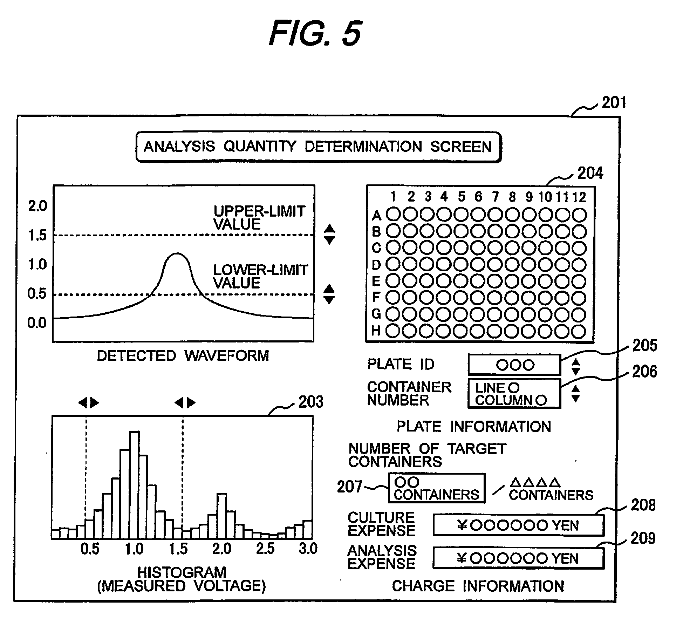

When culture is necessary, the user accesses the server 132, and then the analysis quantity determination screen 201 appears as shown in FIG. 5. Whether the container, which is considered to contain only one microorganism, actually contains only one microorganism is closely examined by checking the waveform measured at the separation operation, and containers, which contain two or more microorganisms or contain impurities smaller than a microorganism, are rejected. The analysis quantity determination screen 201 displays the detected waveform, histogram of the measured voltage of the absorbance, plate ID which is an identification indicator 48 for identifying the plate, receiver number (line and column showing the position on the plate), quantity of the receiver, culture expense, and analysis expense.

The detected waveform 202 at the upper left on the screen 201 shows the waveform detected by a sensor when each container was separated, and the histogram 203 at the bottom left on the screen 201 shows the peak height of all detected waveforms in the form of a histogram. For example, in the case of voltage measurement, with regard to the detected waveform to be shown, changes of measured voltage due to changes of resistance when a microorganism passes through are stored as data.

The height of the detected waveform is almost proportional to the volume of an object that passed by the sensor. Therefore, when the intention is for only one microorganism to pass, setting an upper-limit value and a lower-limit value of the height of the waveform will reveal a waveform that is twice the upper-limit value when two or more microorganisms passed as a lump, and also reveal a waveform lower than the lower-limit value when impurities smaller than a microorganism passed.

A user determines which container should be cultured by using two kinds of data: detected waveform and histogram. If the lower-limit value for determination is set too low, a container which contains impurities smaller than a microorganism is to be cultured, and if the upper-limit value is set too large, a container which contains two or more microorganisms as a lump is to be cultured. This means that the number of containers to be cultured changes according to the settings of the upper-limit value and the lower-limit value which are threshold values.

By changing two threshold values, the number of target containers is calculated in real time in the server 132 and displayed at the bottom right on the screen 201. Furthermore, both expense 208 necessary for culture and expense 209 necessary for analysis are calculated in the server 132 and displayed on the screen 201. Expense for analysis or recovery depends on the number of target containers. By limiting the threshold values, the number of target containers decreases thereby decreasing the expense, and by increasing the threshold values, the number of target containers increases thereby increasing the expense.

Plate information 204 at the upper right on the screen 201 shows the target, colored container. By changing a plate ID 205 or a container number 206, it is possible to check all of the waveforms. Based on the results, a user determines the upper-limit value and the lower-limit value.

It is possible to automatically determine the threshold values by using the histogram. Because the histogram shows a maximum value and a minimum value, it is easy for the histogram to recognize the size of a microorganism. Therefore, it is possible to automatically determine threshold values based on the data.

Next, the user confirms the analysis quantity determination screen 201 and indicates a receiver, and then the indicated receiver is to be cultured. It is desirable that the space for culture be adjacent to the separating apparatus from the viewpoint of contamination by unwanted bacteria, and as the traveling distance of the receiver becomes shorter, the environment changes less. Therefore, it is desirable that the separating apparatus, analyzing apparatus, and the culture apparatus should be placed in a connected non-bacteria space, for example, a temperature-controlled room.

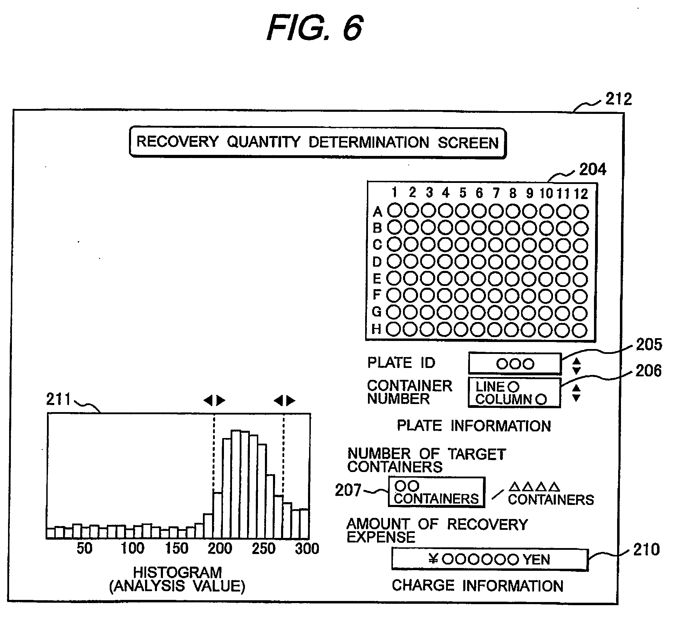

When analysis is not necessary, the cultured microorganism is to be delivered, and when analysis is necessary, analysis of the target receiver is to be carried out. As an analysis method, as far as the method will not kill microorganisms, it is possible to use all of the cultured bacteria. However, if an analysis will kill microorganisms, the minimum necessary amount of microorganisms and its culture liquid are to be taken out from the target container and analyzed. Analytical results are stored in the server 132. After that, the user accesses the server 132 and browses the recovery quantity determination screen 212 shown in FIG. 6. At the bottom left on the screen 212, the histogram 211 of the analytical values are shown, and the range of the target microorganism analysis values will be determined. Analytical results are the measurement results, such as absorbance, which change according to the amount of the existing microorganisms. Or, for example, it is possible to introduce a reagent that reacts with chemical species discharged as metabolite from a target microorganism, thereby knowing the existence of the target microorganism and storing the result as data.

As the threshold values change, the server 132 calculates the number of target containers 207 and shows it at the bottom right on the screen 212. The expense 210 necessary for recovery is also displayed. The plate information 204 displays a value based on the analytical result or a value converted into color information. After the threshold values have been determined, an operator recovers microorganisms and delivers the microorganisms, which have been separated and analyzed, to the user.

As stated above, the number of containers to be used in the next step is determined based on the separation results and analytical results, it is possible to reduce chemicals to be used and analysis time thereby reducing expenses. Furthermore, for example, on the operator side, a series of procedures from separation to culture can be conducted in a closed space free of unwanted bacteria, such as a temperature-controlled room. Thus, it is easy to prevent unwanted bacteria from entering, thereby maintaining the optimal culture environment and increasing the probability of obtaining pure bacteria. Moreover, unnecessary containers will not be delivered to the user and the traveling of the container from the separation procedure to the end of analysis can be minimized; therefore, it is possible to increase efficiency and minimize changes of the microorganism culture environment.

Furthermore, when microorganisms are brought in from overseas, it is easy to pass a quarantine inspection at the import if the properties of the microorganisms are well understood. Therefore, instead of bringing in soils and water that may contain microorganisms, it is desirable that the microorganisms be separated and analyzed on site and their properties be understood. And it is also possible to check separation results and analytical results via a network. Therefore, it is possible to remotely operate criteria for the separation results and analytical results, thereby making it possible to efficiently acquire useful microorganisms.

## Claims

1. Microorganism separation system comprising
a sample solution container (34) containing a sample solution including microorganisms,
a separator (1) having a first flow channel (2) and a second flow channel (10),
a pump (34) for supplying the sample solution in the sample solution container (34) to the first flow channel (2),
a carrier liquid container (44) containing a carrier liquid,
a carrier liquid pump (42) for supplying the carrier liquid in the carrier liquid container (44) to the second flow channel (10), and
a receiver (47) for receiving the carrier liquid containing the sample solution discharged from the end portion of the second flow channel (10), designed to separate microorganisms dispersed in the sample solution;
further comprising
a microorganism detection sensor (55, 7, 8) located between the end portion of the first flow channel (2) and the second flow channel (10) and emitting a detection signal when a microorganism passes, and
a plate (49) having a plurality of the receivers (47) connected to each other and an identification indicator (48), wherein
when the microorganism detection sensor (55, 7, 8) determines that a microorganism has passed, supply of the sample solution to the first flow channel (2) is stopped, the detected microorganism is discharged together with the sample solution from the end portion of the second flow channel (10), and then the solution starts to be injected into the receiver (47), and the number of times microorganisms are detected during the time period from the start of the injection to the end is recognized as separation quantity, and then, as separation information, are stored:
the separation quantity, and/or the number for the receiver (47) into which a microorganism was injected for each identification by the identification indicator (48),
the signal waveform sent from the microorganism detection sensor (55, 7, 8) at the separation procedure, the date and the temperature.

2. Microorganism separation system according to claim 1, further comprising
a server (132) for storing the separation information, and
an analyzing apparatus (111) connected to the server (132) via a network (131) for analyzing the contents of the receiver (47), wherein
the separation information and the values measured by the analyzing apparatus (111) can be browsed.

3. Microorganism separation system according to claim 1, further comprising
a server (132) for storing the separation information, and
an analyzing apparatus (111) connected to the
server (132) via a network (131) for analyzing the contents of the receiver (47), wherein
the separation information and the values measured by the analyzing apparatus (111) are stored in the server (132) and can be browsed via the Internet (134).

4. Microorganism separation system according to claim 1, further comprising
a server (132) for storing the separation information, and
an analyzing apparatus (111) connected to the server (132) via a network (131) for measuring the absorbance of the receiver (47), wherein
the separation information and the values measured by the analyzing apparatus (111) are stored in the server (132).

5. Microorganism separation system according to claim 4, wherein
the separation information and the values measured by the analyzing apparatus (111) are stored in the server (132), and an analysis quantity determination screen (201) appears on a terminal connected to the server (132) and displays the signal waveform from the microorganism detection sensor (55, 7, 8), the histogram of the absorbance, the identification of the identification indicator (48), the number of the receiver (47), the quantity of the receiver (47), the culture expense, or the analysis expense.

6. The microorganism separation system according to any of claims 1 to 5, further comprising
a server (132) for storing the separation information, an analyzing apparatus (111) connected to the server (132) via a network (131) for measuring the absorbance in the receiver (47), and a culture apparatus for culturing microorganisms by using the receiver (47), wherein
the separation information and the values measured by the analyzing apparatus (111) are stored in the server (132), and an instruction is sent from a terminal connected to the server so as to indicate the receiver (47) thereby culturing the microorganism.

7. The microorganism separation system according to any of claims 1 to 6, wherein
the separating apparatus (101), the analyzing apparatus (111), and the culture apparatus are located in a continuously connected bacteria-free space.

8. Method for separating microorganisms dispersed in a sample solution in a microorganism separation system comprising a sample solution container (34) containing the sample solution including microorganisms,
a separator (1) having a first flow channel (2) and a second flow channel (10),
a pump (34) for supplying the sample solution in the sample solution container (34) to the first flow channel (2),
a carrier liquid container (44) containing a carrier liquid,
a carrier liquid pump (42) for supplying the carrier liquid in the carrier liquid container (44) to the second flow channel (10), and
a receiver (47) for receiving the carrier liquid containing the sample solution discharged from the end portion of the second flow channel (10), designed to separate microorganisms dispersed in the sample solution,
a microorganism detection sensor (55, 7, 8) located between the end portion of the first flow channel (2) and the second flow channel (10) and emitting a detection signal when a microorganism passes, and
a plate (49) having a plurality of the receivers (47) connected to each other and an identification indicator (48),
**characterized by**:
- introducing the sample solution from the sample solution container (34) into the first flow channel (2) of the separator (1),
- continuously monitoring a physical property of the sample solution with the microorganism detection sensor (55, 7, 8), preferably the electrical resistance,
- detecting the passage of a microorganism through an orifice (60) at the end of the first flow channel (2) by a change of the signal of the microorganism detection sensor (55, 7, 8),
- stopping the supply of sample solution by stopping the pump (32) if a microorganism has passed through the orifice (60) and resides in the second flow channel (10), and
- operating the carrier liquid pump (42) and injecting the carrier liquid containing the microorganism into a receiver (47),
- repeating these steps for injecting carrier liquid containing microorganisms one by one into a plurality of containers (47),
and
- detecting the number of times microorganisms have been detected during the time period from the start of the introduction of the sample solution to the stopping of the sample solution supply to the first flow channel (2), which corresponds to the number of separated microorganisms that were dispersed into a respective receiver (47) (separation quantity),
- rejecting containers (47) containing more than one microorganism or contain impurities smaller than a microorganism by checking the signal waveforms detected at the respective separation operation, and using one container (47) containing only one single microorganism for analysis and/or recovery.

9. Method according to claim 8, **characterized in that** the separated single microorganisms in containers (47) are analyzed.

10. Method according to claim 9, **characterized in that** the analysis is made in an analyzing apparatus (111) by measuring the absorbance, the degree of fluorescence, the pH value, the redox potential, the COD value, the BOD value, the TOC value, the dissolved oxygen concentration of a culture liquid, by staining with a staining reagent, and/or by measuring a metabolite of the microorganism directly or indirectly using a reagent.

11. Method according to any of claims 8 to 10, **characterized in that** the separated microorganisms are cultured before or after the analysis.

12. Method according to any of claims 8 to 11, **characterized in that** for the selection of containers (47) containing only one specific microorganism a histogram is used, and the selection is made using an upper and a lower limit value derived from the histogram.

13. Method according to any of claims 8 to 12, **characterized in that** for the determination of the recovery quantity a histogram is used.

14. Method according to any of claims 8 to 13, **characterized in that** the separation information and the analysis information are stored in a server (132) of a network (131, LAN), the server (132) preferably being connected to the Internet (134) and allowing browsing separation and analysis data.
